(19) Europäisches Patentamt
European Patent Office
Office européen des brevets

(11) Publication number: **0 423 641 A2**

## EUROPEAN PATENT APPLICATION

(21) Application number: 90119603.0

(22) Date of filing: 12.10.90

(51) Int. Cl.⁵: **C12N 15/70**, C12N 15/62, C12N 1/21, //(C12N1/21, C12R1:19)

(30) Priority: 18.10.89 JP 271249/89
18.10.89 JP 271250/89

(43) Date of publication of application:
24.04.91 Bulletin 91/17

(84) Designated Contracting States:
DE FR GB IT

(71) Applicant: NIPPON MINING COMPANY LIMITED
10-1, Toranamon 2-chome
Minato-ku Tokyo(JP)

(72) Inventor: Misawa, Satoru

17-35, Niizominami 3-chome
Toda-shi, Saitama(JP)
Inventor: Shintani, Makoto
17-35, Niizominami 3-chome
Toda-shi, Saitama(JP)
Inventor: Matsuda, Hitoshi
17-35, Niizominami 3-chome
Toda-shi, Saitama(JP)

(74) Representative: Boeters, Hans Dietrich, Dr. et al
Boeters & Bauer Bereiteranger 15
W-8000 München 90(DE)

(54) Expression vector, transformed microorganism, fusion protein and method for the production of platelet factor 4 or TGFalpha.

(57) A method utilizing gene technology for the production of platelet factor 4 or TGFα employing an expression vector which includes DNA fragments of tac promotor region, replication origin of pUC18, and DNA coding all or a part of amino acid sequence of human angiogenin or porcine adenylate kinase and DNA coding amino acid sequence of platelet factor 4 or TGFα, and is designed to produce a fusion protein.

Glu Ala Glu Glu Asp Gly Asp Leu Gln Cys Leu Cys Val Lys

Thr Thr Ser Gln Val Arg Pro Arg His Ile Thr Ser Leu Glu Val Ile Lys Ala

Gly Pro His Cys Pro Thr Ala Gln Leu Ile Ala Thr Leu Lys Asp Gly Arg Lys

Ile Cys Leu Asp Leu Gln Ala Pro Leu Tyr Lys Lys Ile Ile Lys Lys Leu Leu

Glu Ser

Fig. 1

**EXPRESSION VECTOR, TRANSFORMED MICROORGANISM, FUSION PROTEIN AND METHOD FOR THE PRODUCTION OF PLATELET FACTOR 4 OR TGFα.**

FIELD OF THE INVENTION

The present inenvention relates to an method for the production of platelet factor 4 or TFGα utilizing gene technology, an expression vector, a microorganism transformed by introducing the vector into Escherichia coli , and fusion protein which is produced by the microorganism.

DESCRIPTION OF THE PRIOR ART

Thus fur, many polypeptides have been identified from human body and it has been elucidated that these polypeptides are responsible for physiologically important role. Among them, platelet factor 4 is a polypeptide which was identified in human platelet and has characteristics that this factor inhibits the proliferation of tumor cells without affecting the growth of normal cells.

This platelet factor 4, called oncostatin A in other way, is composed of 60 amino acids and its molecular weight is about 7,000. Amino acid sequence has already been determined and the existance of several variants of platelet factor 4 has been clarified (USP. No.4,590,003, No.4,645,828, No4,737,580).

These compounds can be administrated to human and applied for the prevention, diagnosis and therapy of cancer since these compounds can inhibit the growth of tumor cells without affecting that of normal cells.

TGFα (transforming growth factor type α) was orignally reported to exist in the culture supernatant of rat cells which was transformed with sarcoma virus (J. Todaro et al. Nature, 264 , 26-31 (1976)).

It is known that TGFα has close relationship with epidermal growth factor(EGF) in terms of biological activities and binding to EGF receptor. Moreover, TGFα has common biological activities with EGF such as stimulation of DNA synthesis, enhancement of the formation of colonies in soft agar and opening of eyelids of newborn mice. TGFα, on the other hand, shows higher bone absorption activity and angiogenic potency than EGF, and therfore is expected to be utilized as a drug like EGF.

On the structure of TGFα, firstly TGFα was isolated and purified from culture supernatant of rat fibroblast transformed with sarcoma virus, and then amino acid sequence was determined.

Next, human TGFα precursor gene was isolated from the human gene library. It was elucidated that human TGFα is a polypeptide consisting from 50 amino acids as shown below ;

Val-Val-Ser-His-Phe-Asn-Asp-Cys-Pro-Asp-Ser-His-Thr-Gln-Phe-Cys-Phe-His-Gly-Thr-Cys-Arg-Phe-Leu-Val-Gln-Glu-Asp-Lys-Pro-Ala-Cys-Val-Cys-His-Ser-Gly-Tyr-Val-Gly-Ala-Arg-Cys-Glu-His-Ala-Asp-Leu-Leu-Ala

Only 4 amino acids as indicated below, are different between human and rat TGFα. Moreover, it is known that there are several variants in human TGFα.

| position | human TGFα | rat TGFα |
|----------|-----------|----------|
| 7 | Asp | Lys |
| 15 | Phe | Tyr |
| 28 | Asp | Glu |
| 41 | Ala | Val |

In order to utilize platelet factor 4 and TGFα as a drug for therapeutic use and diagnosis, it is prerequisit to obtain in a large quantity without loosing biological activities. However, it is extremely difficult to provide a large quantity of platelet factor 4 from human platelet and it requires enormous labor to collect large quantity of TGFα from the culture medium of rat cells transformed with sarcoma virus, and therefore, it is not effective. Several attempts to produce these polypeptides by gene technology have been proposed (for example, USP. No.4,590,003, No.4,645,828 No.4,737,580 and USP.No.4,742,003, Japanese Laid-open No. 63-313586 and so on for TGFα), however, a satisfactory result has not been obtained even by this technique.

2

## SUMMARY OF THE INVENTION

It is an object of the present invention to provide a method utilizing gene technology to produce platelet factor 4 or TGFα in a large quantity with high efficiency.

It is also an object of the present invention to provide expression vectors which can express platelet factor 4 or TGFα as a fusion protein.

Another object of the present invention is to provide a transformed micro-organism (Escherichia coli ) to which the aove-mentioned vectors are introduced.

A further object of the present invention is to provide fusion proteins which contain platelet factor 4 or TGFα.

These objects of the present invention can be achieved by providing production methods for expression vectors, transformed microorganisms, fusion proteins, and platelet factor 4 or TGFα. These are ;

(1) An expression vector for a polypeptide chosen from a group consisting from platelet factor 4 and TGFα, which contains DNA fragments containing a tac promoter region, replication origin (Ori) of plasmid pUC18, nucleotide sequence coding all or part of amino acid sequence of a polypeptide chosen from a group comsisting from human angiogenin and porcine adenylate kinase, and a nucleotide sequence of a polypeptide chosen from a group consisting from platelet factor 4 and TGFα, and which is designed to express a fusion protein comprising all or a part of polypeptide chosen from a group consisting from human angiogenin and porcine adenylate kinase and a polypeptide chosen from a group consisting from plateletfactor 4 and TGFα.

(2) A fusion protein comprising all or a part of amino acid sequence of a polypeptide chosen from a group consisting from human angiogenin and porcine adenylate kinase and that chosen from a group consisting from platelet factor 4 and TGFα.

(3) A transformed microorganism which is made by introducing the expression vector mentioned above.

(4) A method for the production of platelet factor 4, TGFα or their variants in which the above mentioned transformed microorganism is cultured, the fusion protein is expressed, collected, and cleaved, and a polypeptide or its variant chosen from a group consisting from platelet factor 4 and TGFα is recovered.

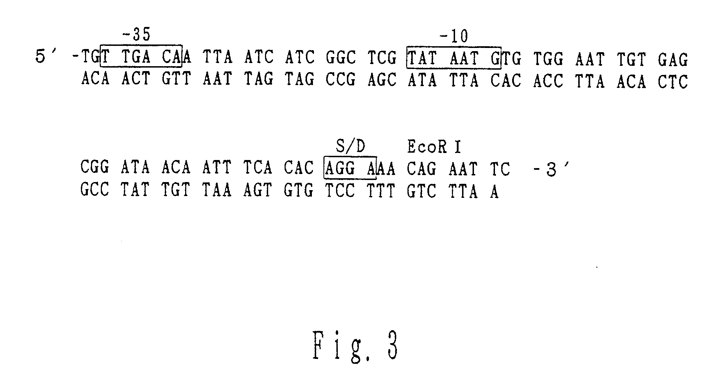

First, the method to produce platelet factor 4 will be explained hereinafter. Tac promotor region mentioned in the present invention have the nucleotide seqence shown in Fig. 3 and can be easily synthesized using a DNA synthsizer and so on. Therefore, the synthesized promotor region can be joined with the replication origin (Ori) of plasmid pUC18. However, commercially available plasmids can be used for the vector construction. For example, PvuI-NruI fragment of plasmid pKK223-3 (Pharmacia AB, catalogue No. 27-4935-01) which contains tac promotor region and PvuI-NruI fragment of plasmid pUC18-(for example Pharmacia AB, catalogue No 27-4949-01) are combined using T4 DNA ligase to yield plasmid pMK2. Then, all or a part of DNA of human angiogenin is inserted into the plasmid. Thus, a vector which comprises the DNA fragments containing tac promotor region, replicatin origin (Ori) derived from plasmid pUC18, and all or a part of DNA of human angiogenin, can be obtained. The vector in which all of human angiogenin gene is inserted has been proposed by the present inventors as human angiogenin expression vector pMAGN1 (Japanese Laid-open No. 1-212442). The expression vector for platelet factor 4 of the present invention can be relatively easily constructed by the use of this expression vector.

In the present invention, the above mentioned vector comprising fragments containing tac promotor region, replication origin (Ori) derived from plasmid pUC18, and all or a part of human angiogenin coding nucleotide sequence may be used. There is no obstacle to employ a vector which contains other genes than the genes included in the plasmid described herein. For example, it is particularly desirable to use a vector which contains the fragments of rrnB terminator, ampicillin-resistance gene and so on, because high expression can be achieved and screening is easy.

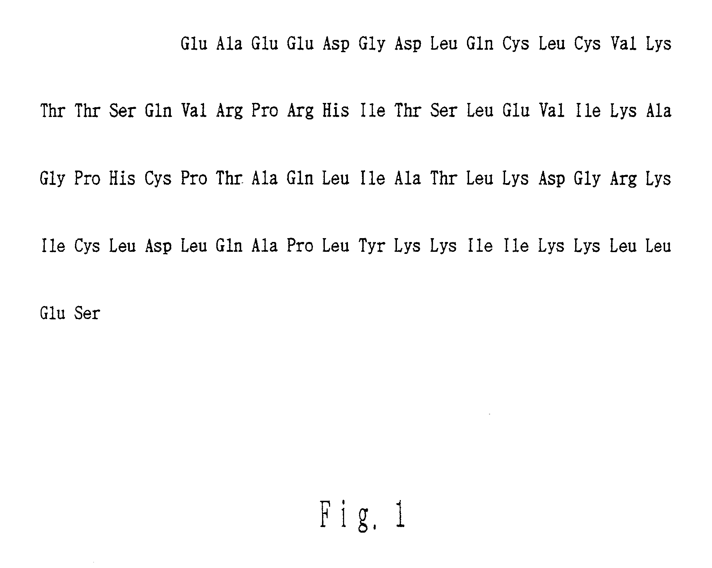

On the other hand, platelet factor 4 gene can be relatively easily synthesized by a DNA synthesizer like human angiogenin gene, since the number of amino acids composing platelet factor 4 is 70, which is relatively small, as shown in Fig.1.

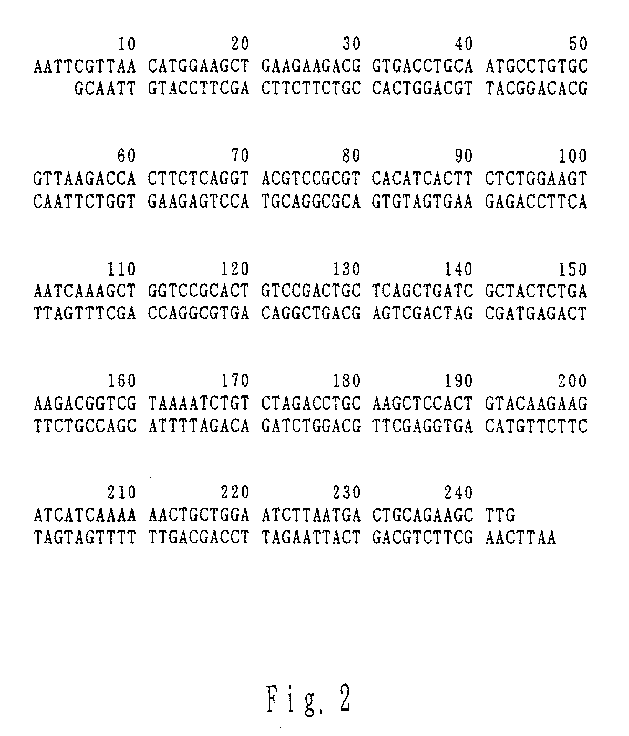

There is no obstacle, of course, to obtain cDNA by screening cDNA library. It is desirable to employ codons which are frequently found in Ecsherichia coli in designing such a gene. Especially, the gene which has the nucleotide sequence indicated in Fig.2 is desirable.

Next, the above mentioned platelet factor 4 gene is inserted into the vector comprising DNA fragments containing tac promotor region, replication origin (Ori) of plasmid pUC18, and DNA comprising nucleotide sequence coding all or a part of amino acid sequence of human angiogenin. Insertion of the gene can be done by the digestion of the vector with restriction enzymes. In the case plasmid pMAGN1 is used as the above mentioned vector, NruI is desirable to cleave the vector and platelet factor 4 gene is joined. Several methods which have been already known are available. Among them, it is especially desirable to combine

EP 0 423 641 A2

the plasmid and the DNA fragment after the removal of 5′ phosphate residue from the NruI cleavage site within the plasmid pMAGN1 by the digestion with alkaline phosphatase and bluntending of both ends of platelet factor 4 gene with Klenow enzyme. In this method, platelet factor 4 gene fragment is combined to the tail of nucleotide sequence coding the 66th amino acid of human angiogenin and this enables the expression of fusion protein in a large quantity.

Thus the expression vector pMAPF4 for platelet factor 4 fusion protein can be obtained.

Furthermore, transformed microorganism can be obtained by introducing the expression vector for platelet factor 4 fusion protein into Escherichia coli and then screening.

Platelet factor 4 fusion protein is produced by the culture of transformed microorganism in the appropriate medium and then, the cells are destroyed and the fusion protein is isolated and purified using the techniques of centrifugation, column chromatography and so on.

The fusion protein is cleaved by bromocyanamide treatment and so on and platelet factor 4 is obtained.

The present invention includes the expression of platelet factor 4 as well as its variants.

Next, the method to obtain TGFα will be described hereinafter.

As for TGFα, human TGFα, rat TGFα and so on are known as mentioned before. Subtypes of human TGFα are also known to exist and variants of TGFα of which a part of amino acids are substituted with other amino acids or deleted but possesing same biological activities (references as mentioned above and Japanese Laid-open No. 63-316798). The present invention is applied to these variants and TGFα which is described in the present invention includes these variants.

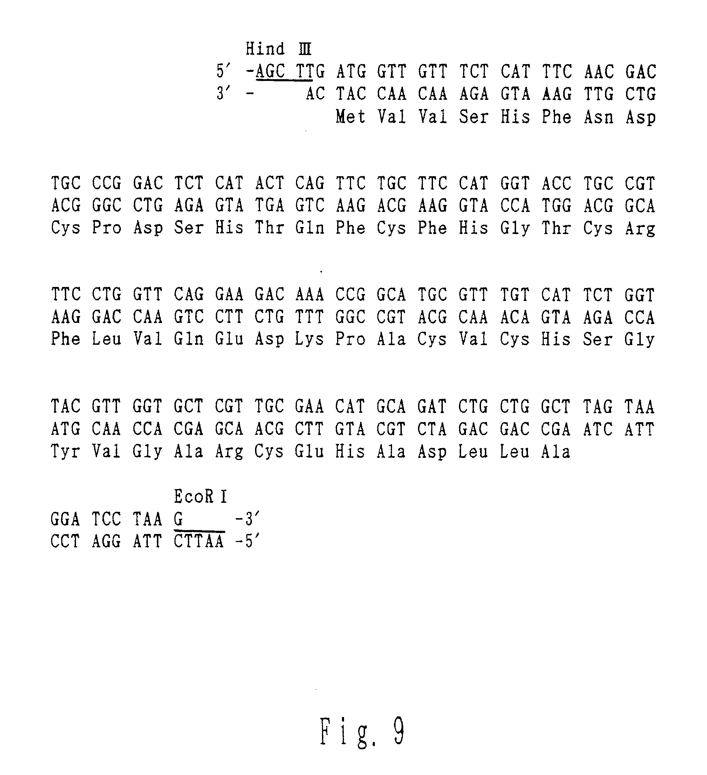

Because the number of amino acids composing TGFα is 60, which is relatively small, TGFα gene of the present invention can be relatively easily synthesized by a DNA synthesizer like platelet factor 4. It is, of course, possible to obtain TGFα cDNA by screening cDNA library. It is desirable to use codons which are frequently found in Escherichi coli . Especially the gene, which has such a nucleotide sequence that can be ligated with DNA fragments which are cleaved with restriction enzymes HindIII and EcoRI as indicated in Fig. 9. is preferable. Then, this gene is inserted into plasmid, trasfected into Escherichia coli , proriferated, and the gene may be cleaved from the plasmid with restriction enzymes.

Any plasmids which are able to amplify TGFα gene can be used, however, it is desirable to use plasmid pEX14 prepared as follows ;

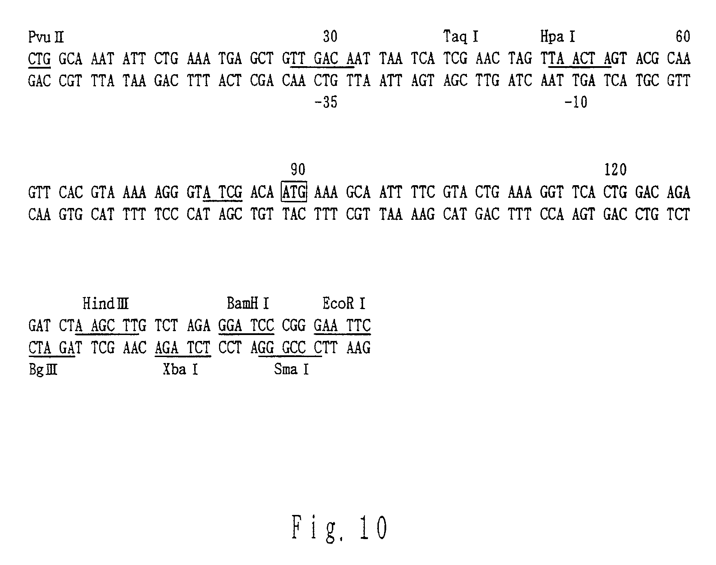

Commercially available plasmid pBR322 (for example Pharmacia AB, catalogue No 27-4902) is cleaved between EcoRI and PvuII site and joined with DNA containing nucleotide sequence indicated in Fig. 10 which comprises DNA fragments containing ampicillin-resistance gene (Apr) and replication origin (Ori), tryptophan promotor region, a part of structural gene coding tryptophan LE protein (C. Yanofski et al. Nucleic Acid Research 9 6647-6659) and a cloning site. Thus, plasmid pEX14 is obtained.

This plsmid is cleaved with restriction enzymes, preferablly. EcoRI and HindIII, and TGFα gene is inserted herein and ligated using T4 DNA ligase. This plasmid pTZT is transfected into Escherichia coli and allowed to proliferate. Then the plasmid is recovered from Escherichia coli and the fragment of TGFα cleaved with EcoRI and HindIII may be used.

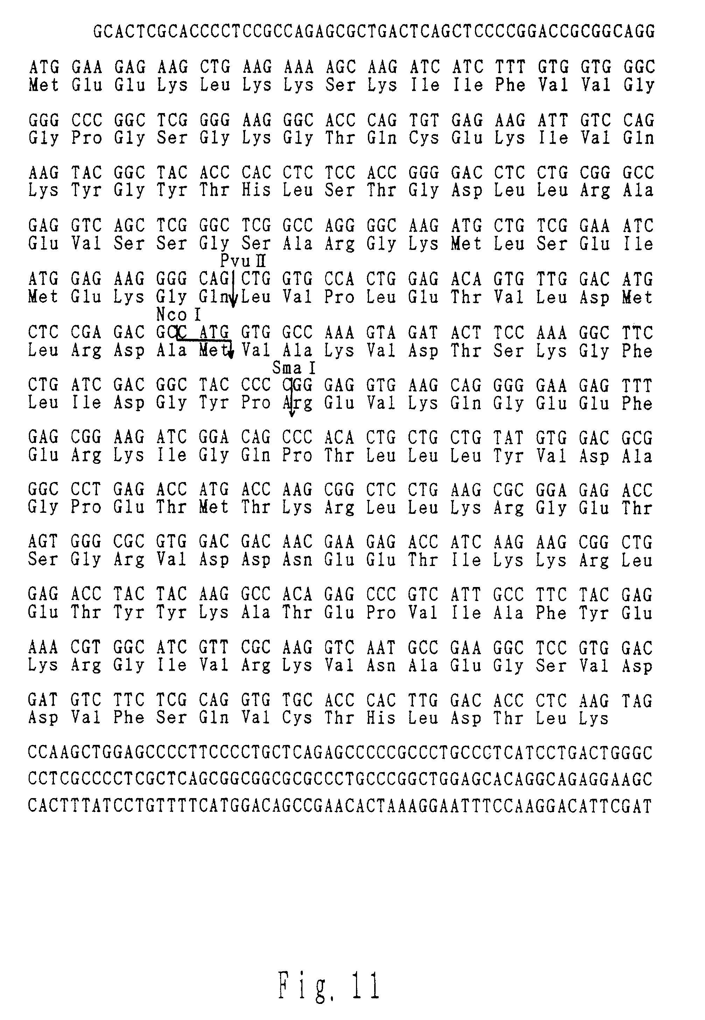

On the other hand, tac promotor fragment which includes nucleotide sequence indicated in Fig. 3 , can be easily synthesized by a DNA synthsizer and so on as mentioned above. Accordingly, these promotors may be synthesized and combined with fragments of replication origin (Ori) of plasmid pUC18. However, this is relatively easily prepared by comibining the cleavage and ligation of commercially available plasmids. For example, plasmid pMK2 is obtained by combining, using T4 DNA ligase, a site region which includes tac promotor region cleaved from commercially available plasmid pKK 223-3(for example, Pharmacia AB, catalogue No. 27-4935-01) with restriction enzymes PvuI and NruI, and a site region which includes replication origin (Ori) fragment cleaved from commercially available plasmid pUC18 (for example, Pharmacia AB, catalogue No. 27-4949-01) with restriction enzymes PvuI and NruI. Then, the vector containing DNA fragments of tac promotor region, replication origin (Ori), DNA comprising nucleotide sequence coding all or a part of amino acid sequence of pcrcine adenylate kinase is obtained, when all or a part of porcine adenylate kinase DNA is inserted into plasmid pMK2. Of these vectors, a vector which is inserted withDNA coding porcine adenylate kinase and nucleotide sequence indicated in Fig. 11, has already been proposedby the present inventors as a porcine adenylate kinase expression vector (Japanese Laid-open No. 64-51088). Byusing this expression vector, TGFα expression vector ofthe present invention can be relatively easily constructed.

In the present invention, a vector including DNA fragments of tac promotor region, replication origin (Ori) of plasmid pUC18 and DNA fragment comprising nucleotide sequence coding all or a part of amino acid sequence of porcine adenylate kinase may be used, however there is no obstacle to employ a vector containing other genes than the genes included in the. plasmid described herein.For example, it is particularly desirable to use a vector. which contains the DNA fragment of rrnB terminator, ampicillin-

4

resistance gene and so on, because high expression can be achieved and screening is easy.

Next, TGFα gene is inserted into the vector comprising DNA fragments of tac promotor region, replication origin (Ori) of plasmid pUC18 and DNA consisting from nucleotide sequence coding all or a part of amino acid sequence of porcine adenylate kinase. This insertion can be carried out by the cleavage of the vector with various restriction enzymes. In the case plasmid pMKAK3 is used as a vector, restriction enzyme PvuII is used for the cleavage and TGFα gene is combined. When this enzyme is used, DNA coding the 65th and 66th amino acids of porcine adenylate kinase is cleaved as follows ;

```
        6 5    |   6 6     6 7

        G l n  |   L e u   V a l

        C A G  |   C T G   G T G

        G T C  ↓   G A C   C A C


               P v u I I
```

This plasmid is digested with alkaline phosphatase and 5′ phosphate residue is removed.

On the other hand, fragments of TGFα gene cleaved as mentioned above, is bluntended with Klenow enzyme as shown below, and then inserted into the above mentioned porcine adenylate kinase cleavage site.

```
                M e t   V a l   V a l

        A |  A G C T  T G  A T G  G T T  G T T ... ... ... ... ...  G| A A T T  C



        T   T C G A |A C  T A C  C A A  C A A ... ... ... ... ...  C  T T A A |G



           H i n d I I I     T G F α                                E c o R I
```

By the treatment mentioned above, amino acid sequence after 66th amino acid is not allowed to be expressed as a fusion protein, since the stop codon is included in DNA coding amino acid sequence of TGFα . In the present invention, it is particularly desirable to use plasmid pMAKTGFα obtained as described above as an expression vector.

Moreover, it is desirable to employ an induction-type expressssion vector and construct a plasmid which is suitable for high density culture.

This induction type expression vector is prepared as follows ;

Namely, plasmid pMKIQ, which enables fusion protein gene to be induced with isopropylthiogalactoside (IPTG), is prepared and then DNA fragments of tac promotor region, replication origin (Ori) region and laqIq, a gene with mutationat laqI promotor, of this plasmid can be utilized. Insertion of laqI into the plasmid enables to produce repressor protein in a large quantity, lowers the expression of tac promotor and produces TGFα in a large quantity by adding IPTG to the culture medium after the proliferation of Escherichia coli. is terminated, whereas TGFα is not produced during the proliferation phase.

This plasmid PMKIQ is easily prepared by using commercially available plasmid pTTQ18 and known plasmid PMK2 which was constructed by the present inventors.

Plasmid pTTQ18 is commercially available and its restriction enzyme map has been disclosed (for example, Amersham, code No. rpn 1261).

DNA fragment which contains laqIq of this plasmid pTTQ18 is cleaved with restriction enzymes. Any restriction enzymes which are able to cleave so that cleaved DNA can be joined to plasmid pMK2 and contain laqIq fragment. However,it is generally desirable to use restriction enzymes ScaI and HindIII. Using these enzymes, a DNA fgragment which contains laqIq together with rrnB terminator fragment can be obtained.

On the other hand, the method for preparation, cleavage sites for restriction enzymes and so on are described in detail in Japanese Laid-open No. 64-51088. In the present invention, since DNA fragments of tac promotor region and replication origin (Ori) are utilized, a DNA fragment containing these fragments are cleaved with restriction enzymes. Any restriction enzymes which can cleave the plasmid so that cleaved fragments can contain these fragments and can be combined with fragments from plasmid pTTQ18. However, it is generally desirable to use restriction enzymes ScaI and HindIII, the same enzyms as those used in cleaving plasmid pTTQ18, because this allows to combine each fragment easily. Thus, plasmid pMKIQ is obtained by combining both fragments using T4 DNA ligase. This plasmid contains DNA fragment of ampicillin-resistance gene derived from pMK2. The transformed Escherichia coli with this plasmid is cultured and screening for the ampicillin resistance is carried out. A vector containing these fragments are prepared and its nucleotide sequence is confirmed by Sanger method and so on.

Next, the DNA which expresses TGFα fusion protein is inserted in the plasmid pMKIQ which is obtained as mentioned above. Accordingly, plasmid pMKIQ is cleaved with restriction enzymes.

Any restriction enzymes can be used which are able to cleave DNA so that the cleavage sites of pMKIQ can be combined with DNA comprising nucleotide sequence coding amino acid sequence for TGFα fusion protein. However, EcoRI is generally used. On the other hand, TGFα gene that is combined with porcine adenylate kinase gene is uised. It is particularly desirable to employ plasmid pMAKTGFα in which TGFα gene is inserted between DNA coding 65th amino acid residue and that coding 66th of porcine adenylate kinase. In this case, it is desirable to prepare and use DNA comprising 2 genes, adenylate kinase gene and TGFα gene, connected in tandem using T4 DNA ligase, after digesting plasmid pMAKTGFα with restriction enzymes and isolating fusion protein gene comprising TGFα gene and adenylate kinase gene. It is desirable to use EcoRI as a restriction enzyme. The DNA obtained by this method is inserted into the above mentioned EcoRI digested plasmid PMKIQ. Thus obtained plasmid pIQAKTGFα2 is employed as an expression vector.

Expression vectors, pMAKTGFα and pIQAKTGFα2, are transfected into Escherichia coli JM109 and screening for ampicillin resistance and the increase in the number of copies is carried out. The plasmids cloned in the right direction are named pMAKTGFα and pIQAKTGFα2, respectively.

Escherichia coli transformed with these plasmid is cultured in a appropriate medium and then, TGFα fusion protein is expressed. Cells are destroyed and TGFα fusion protein is isolated and purified using centrifugation, column chromatography, and so on.

This TGFα fusion protein is treated with cyanogen bromide and so on, and finally TGFα is obtained after folding.

The present invention can be applied not only for human TGFα but also for the production of mouse TGFα and so on.

## BRIEF DESCRIPTION OF THE DRAWINGS

Fig. 1 shows amino acid sequence of platelet factor 4.

Fig. 2 shows a desirable example of nucleotide sequence of a DNA fragment between EcoRI and EcoRI of platelet factor 4 gene.

Figs. 3 and 4 show a DNA fragment of tac promotor region and a desirable example of nucleotide sequence for human angiongenin, respectively.

Fig. 5 is a scheme of the process for the construction of human angiogenin gene.

Figs. 6 - 8 inclusive are schemes of the construction process for plasmids pMK2, pMAGN1 and pMAPF 4, respectively.

Fig. 9 shows nucleotide and amino acid sequence of human TGFα gene.

Fig. 10 is a scheme of DNA inserted into plasmid pEX14.

Fig. 11 is a scheme of DNA comprising nucleotide sequence coding amino acid sequence for porcine adenylate kinase and its restriction enzyme cleavage sites.

Figs. 12 - 15 inclusive are schemes of the construction process for plasmids pTZT, pMAKTGFα, pIQAKTGFα2, and pMKIQ, respectively.

## DETAILED DESCRIPTION OF PREFFERED EMBODIMENT

The present invention is shown in detail hereinafter with examples.

(Example 1) Production of platelet factor 4

(1) Construction of plasmid pMAGN1

(i) preparation of human angiogenin DNA

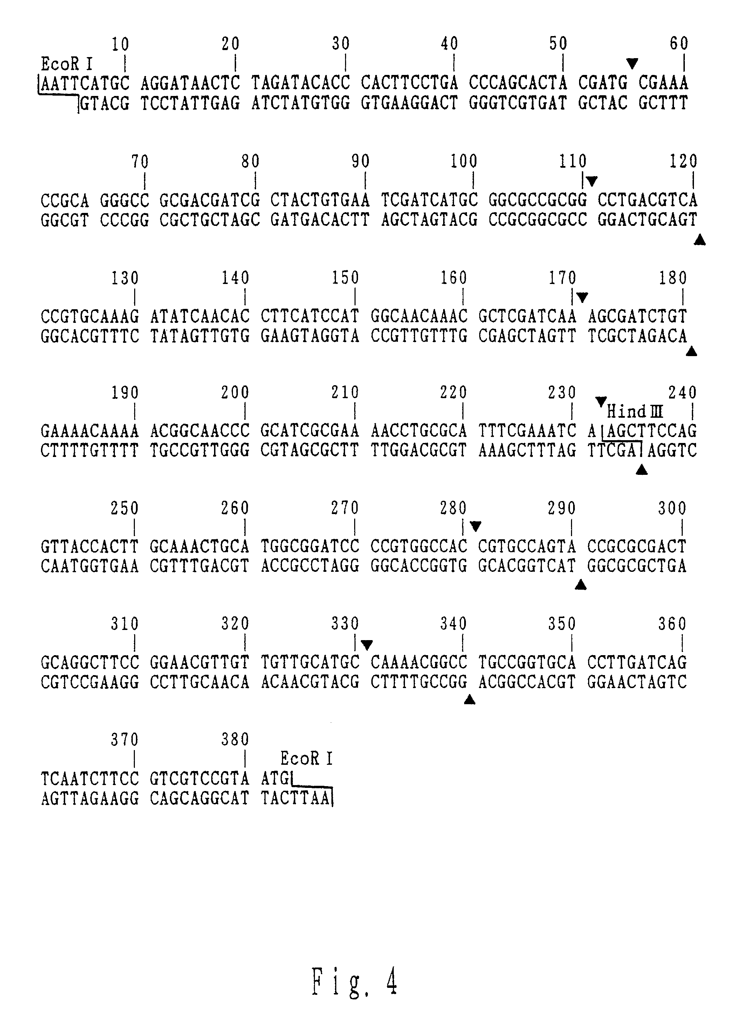

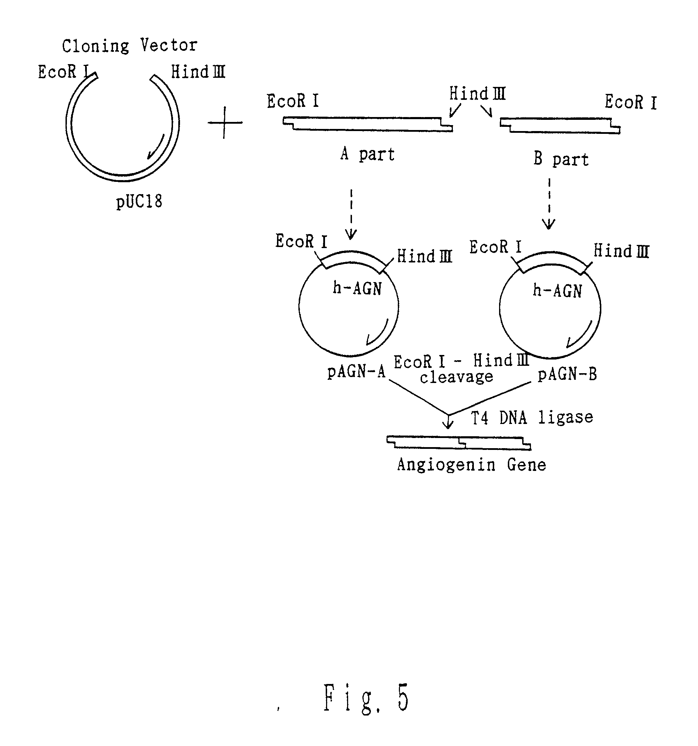

Human angiogenin DNA (shown in Fig.4) is devided into 14 small fragments at the sites indicated by or and each oligo nucleotide fragment was synthesized using a DNA synthesizer (Applied Biosystems Model 380A) and purified using high performance liquide chromatography equiped with a C18 reverse phase column. Each oligo nucleotide (500 pmole) was treated with T4 polynucleotide kinase and 5′ terminus was phospholylated. Next, as shown in Fig 5, these oligonucleotides were separated into 2 parts, the first half (part A) and the latter half (part B) at the HindIII cleavage site (Fig. 4) and oligonucleotides of each part were connected with T4 DNA ligase. Each part was, then, inserted into EcoRI -HindIII site of commercially available expression vector pUC18. Escherichia coli JM109 transformed with this expression vector was allowed to proliferate. Both parts were cleaved from the vector with the above mentioned restriction enzymes and combined using T4 DNA ligase and complete human angiogenin gene was obtained.

(ii) preparation of plasmid pMAGN1

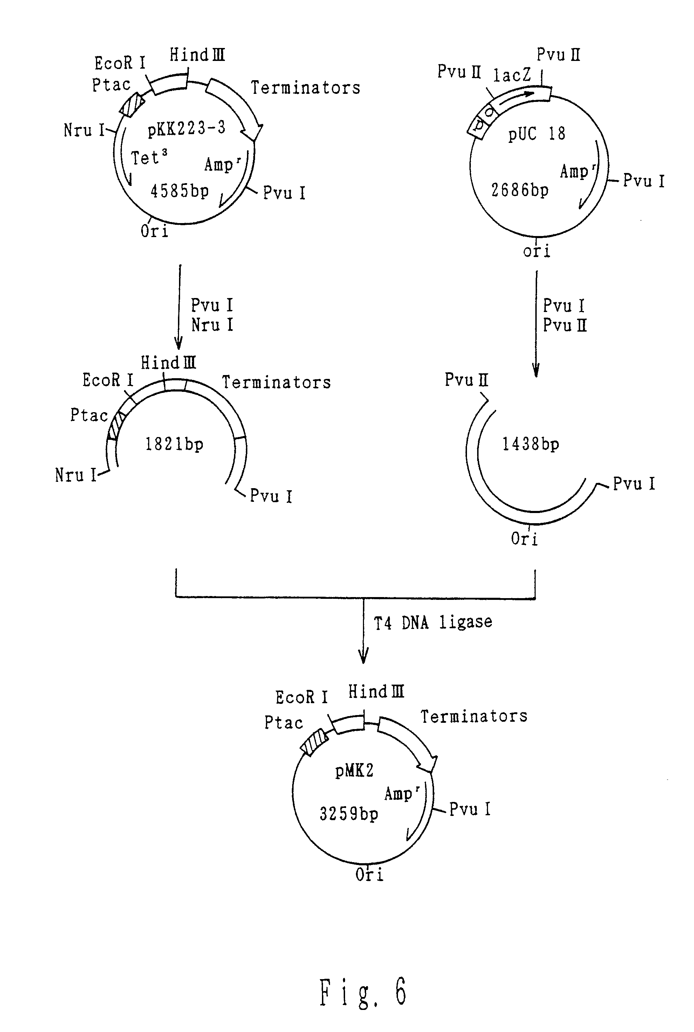

As shown in Fig. 6, a fragment of tac promoter, which was formed by the digestion of commercially available plasmid pKK223-3 (Pharmacia) with restriction enzymes PvuI and NruI, and a fragment containing replication origin (Ori) and ampicilin registant gene, which was cleaved from commercially available plasmid pUC18 were combined using T4 DNA ligase. Escherichia coli JM109 was transfected with this plasmid, cultured, and screened for ampicillin-resistance, and then a vector containing tac promotor of pKK223-3 and replication origin (Ori) of pUC18 was obtained. The nucleotide sequence of this plasmid was confirmed by theSanger method. This plasmid was named pMK2.

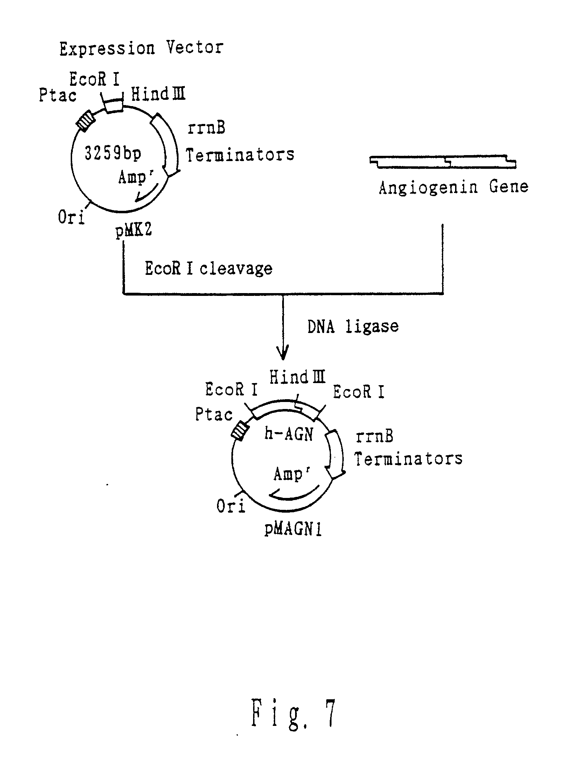

Next, the above mentioned plasmid pMK2 was isolated and digested with EcoRI as shown in Fig. 7. and 5′ phosphate residue was removed by the treatment with alkaline phosphatase. This linear plasmid and human angiogenin gene obtained as described before were combined using T4 DNA ligase. Escherichia coli JM109 was transfected with this plasmid and screened for the ampicillin resistance and the increase in the number of copies. This cloned plasmid was named PMAGN1.

(2) Preparation of plasmid pMAPF4

(i) preparation of platelet factor 4 gene

An oligonucleotide which includes nucleotide sequence coding platelet factor 4 as indicated in Fig. 2 was synthesized using a DNA synthesizer (Applied Biosystems Model 380A) and EcoRI sites of both ends were digested with Klenow enzyme and bluntended as shown below ;

```
          platelet  factor  4  gene              Klenow

      ┌ A A T T    C G T  ┌ A T G ├ - - - - - - - - - G ┌ T T A A
      │                   │                             │
      └ - - - - - - - - - ┐                             └ - - - - - ┐
        T T A A │ C C A    T A C ├ - - - - - - - - C    A A T T │
        ←                                                       ←
       Klenow                   Initiation

                               codon
```

(ii) preparation of plasmid pMAPF4

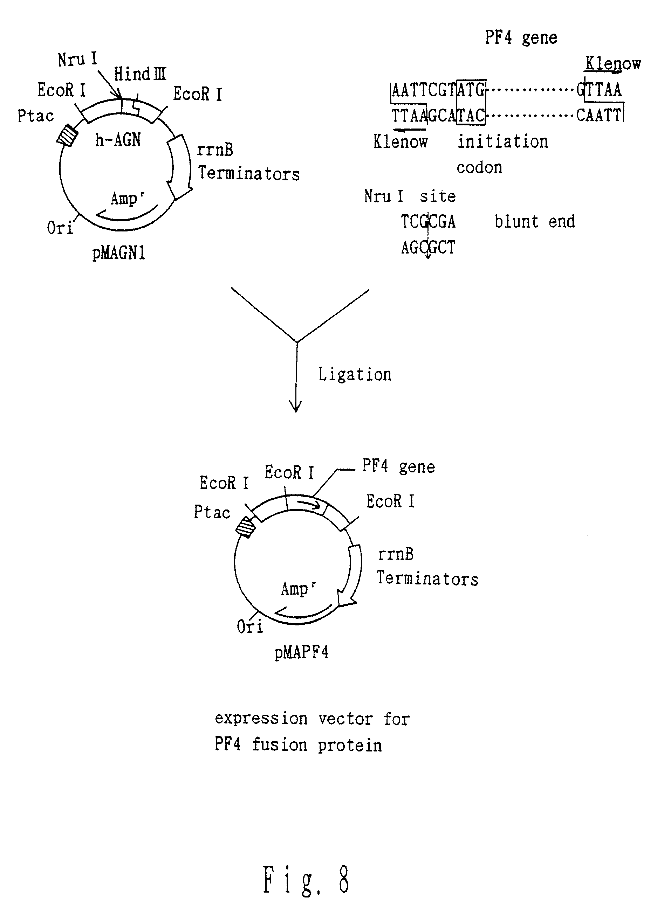

As shown in Fig. 8, DNA fragment which codes amino acid sequence for human angiogenin gene of plasmid pMAGN1 obtained in the above item (1)(ii), was digested with restriction enzyme NruI and its linear DNA was purified by agarose gel electrophoresis. NruI cleavage site was treated with alkaline phosphatase and 5′ phosphate residue was removed and bluntended as shown below ;

$$
\begin{array}{ccc|ccc}
\text{T} & \text{C} & \text{G} & \text{C} & \text{G} & \text{A} \\
\text{A} & \text{G} & \text{C} & \text{G} & \text{C} & \text{T}
\end{array}
$$

Then, the linear plasmid described above was combined with platelet factor 4 bluntended fragment and EcoRI site was generated. Escherichia coli JM109 was transformed with this plasmid and screened for the ampicillin resistance and the increase in the number of copies. This plasmid was named pMAPF4.

(3) Expression of platelet factor 4 fusion protein

Escherichia coli JM109 transformed with the above mentioned plasmid pMAPF4 was cultured in LB medium (Bacto-tryptone 10 g/l, Bacto-yeast extract 5g/l, NaCl 10g/l, pH 7.5) containing 50 μg/ml of ampicillin. After overnight culture, cells were collected by centrifugation, suspended in Laemmli's sample buffer, solubilized by heating and subjected to SDS-polyacryl-amide gel electrophoresis. The gel was stained with Coomassie Brilliant Blue solution and destained and then platelet factor 4 fusion protein was confirmed to be expressing at the position corresponding to molecular weight about 14,000. The amount of platelet factor 4 fusion proteinproduced was about 30% of the total bacterial protein corresponding to the production of 50 mg from 1 l of culture medium.

This strain transfected with plasmid pMAPF4 was deposited to the Fermentation Research Institute as E. Coli JM109 pMAPF4 (FERM BP2610).

Thus obtained platelet factor 4 fusion protein was treated with cyananogen bromide and platelet factor 4 was obtained.

(Example 2) Production of human TGFα

(1) preparation of human TGFα gene

DNA having nucleotide sequence coding human TGFα shown in Fig. 9, was synthesized using a DNA synthesizer (Applied Bioscience Model 380A) by separating into small fragments . Each fragment was annealed and human TGFα structural gene was obtained. Next, commercially available plasmid pBR322 was cleaved at EcoRI and PvuII sites and joined with DNA which has nucleotide sequence indicated in Fig. 10 and comprises ampicillin-resistance gene(Apr), replication origin (Ori), tryptophan promotor region, a part of structural gene coding tryptophan LE protein, and the cloning site. Thus plasmid pEX14 was obtained.

HindIII-EcoRI site was cleaved and small fragment was removed from this plasmid pEX14, and TGFα gene synthesized as described above was inserted using T4 DNA ligase. This vector pTZT was introduced into Escherichia coli JM109 and amplified. Then, plasmid pTZT was isolated and human TGFα gene was cleaved from this plasmid with HindIII and EcoRI.

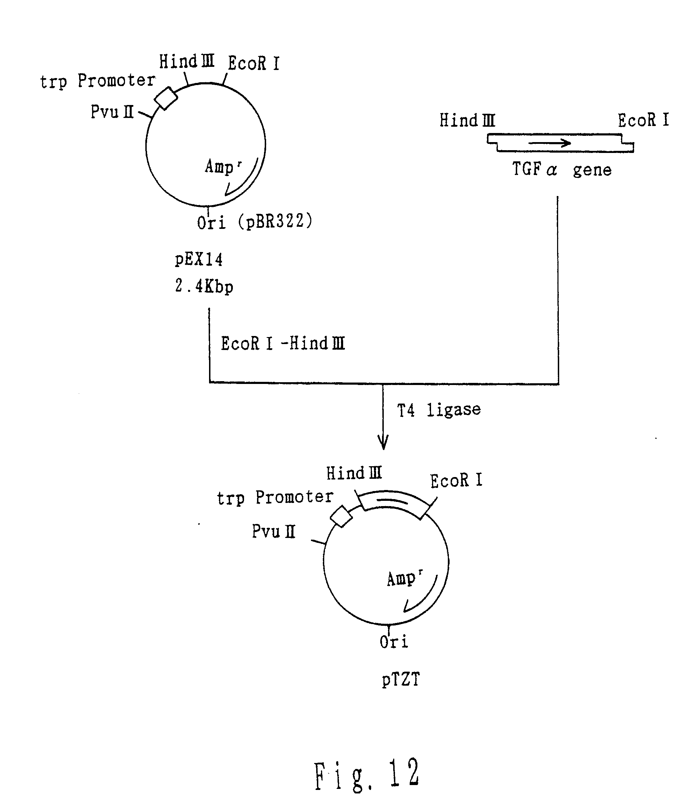

Sanger method was employed to confirm this gene to have the nucleotide sequence indicated in Fig. 9 ( see Fig. 12).

(2) preparation of expression vector

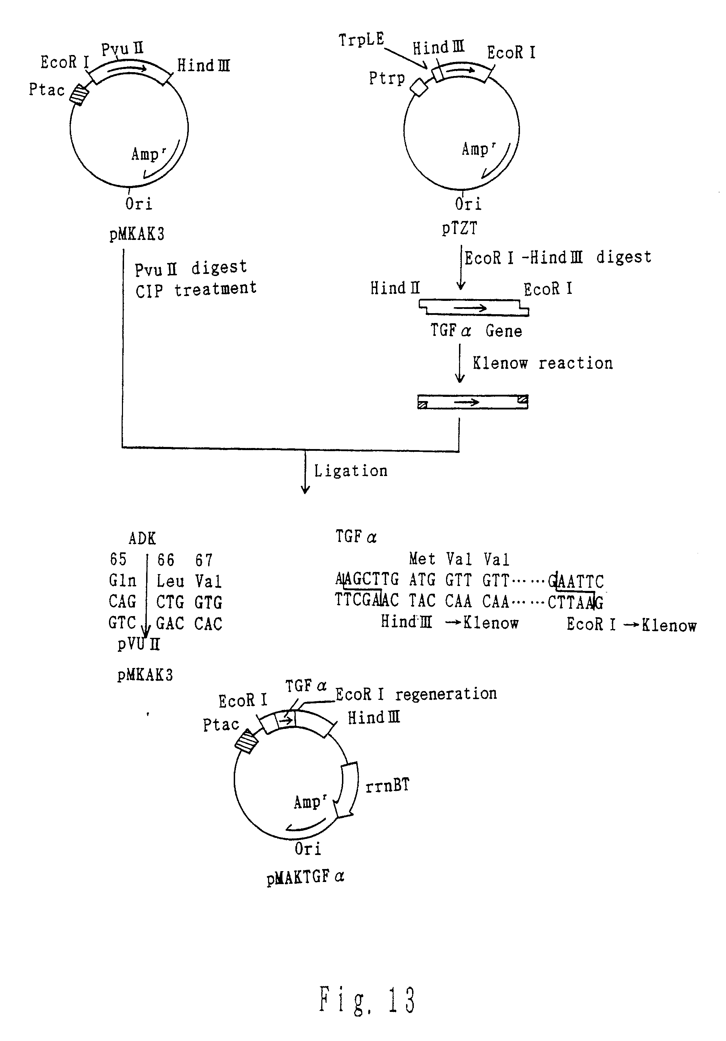

As shown in Fig. 13, plasmid pMKAK3, which was constructed for the expression of porcine adenylate kinase by the present inventors (Japanese Laid-open No 64-51088), was cleaved at the site between the 65th and 66th amino acid of porcine adenylate kinase corresponding Gln and Leu, respectively with restriction enzyme PvuII and 5′ phosphate residue was removed by the treatment with alkaline phosphatase. This plasmid fragment contained tac promotor, replication origin (Ori), ampicillin-resistance gene and so on.

On the other hand, DNA fragment containing TGFα gene which was cleaved with EcoRI and HindIII as described in (1), was bluntended with Klenow enzyme and this fragment was inserted between the cleavage sites of the above mentioned plasmid pMKAK3. This plasmid was introduced into Escherichia coli JM109 and screened for the ampicillin resistance and the increase in the number of copies. This plasmid was named pMAKTGFα.

(3) Expression of human TGFα

Escherichia coli JM109, which was transformed with the above mentioned plasmid, was cultured in 200 ml of LB medium (Bactotryptone 10 g/l, Bacto-yeast extract 5g/l, NaCl 10g/l, pH 7.5) containing 50 μg/ml of ampicillin.

After overnight culture, cells were collected by centrifugation and disrupted and then, the fusion protein comprising polypeptide corresponding to N-terminus - 65th of porcine adenylate kinase and human TGFα was obtained. The expression of this protein was confirmed by SDS-PAGE. This fusion protein was expressed in a large quantity reaching as much as 30% of the total bacterial protein. The fusion protein was treated with cyanogen bromide and TGFα and a part of porcine adenylate kinase were separated. Next, this was desolved in 0.1M Tris-HCl buffer, pH8.6 containing 0.1M ammonium sulphate and 0. 1mM Triton X100 (Rhom &Haas), and glutathion in oxidized and reduced form were added to 0.5 mM and 5.0 mM, respectively. Refolding was carried out for 16 hrs at room temperature and 3 disulfide bonds ( 8th - 21st, 16th - 32nd, 34th - 43rd) were formed.

Next, the protein was dialyzed against 10 mM Tris -HCl buffer, pH8.0 using cellulose tubes(cut MW 1000), lyophilized, and dissolved in 50 mM 4-(2-hydroxyethyl)-1-piperazine-ethane-sulfonate (HEPES) buffer, pH7.7 containing 138 mM NaCl, 1.2 mM KCl and 1.2 mM $Mg_2SO_4$ and purified by high performance liquid chromatography.

Thus, the quantity of TGFα obtained from 1 l of culture medium was 30 mg.

This strain in which plasmid pMAKTGFα introduced was deposited to the Fermentation Research Institute as E. coli JM109 pMAKTGFα with deposition No. FERM BP2611.

(Example 3) Production of human TGFα (2)

(1) preparation of the gene comprising 2 combined human TGFα gene

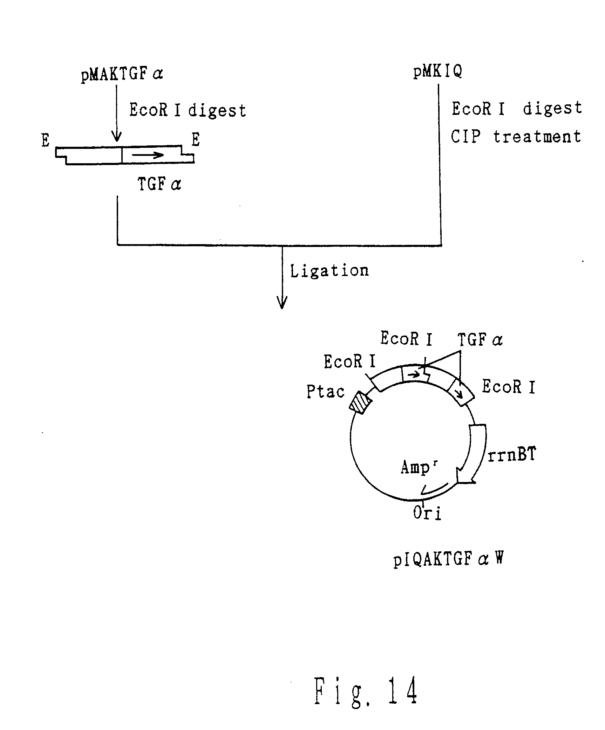

As indicated in Fig. 14, plasmid pMAKTGFα , which was constructed in example 2, was cleaved with restriction enzyme EcoRI, and DNA coding amino acid sequence of the fusion protein comprising N-terminus -65th of porcine adenylate kinase and human TGFα was obtained. Two DNAs were combined using T4 DNA ligase.

(2) preparation of an expression vector

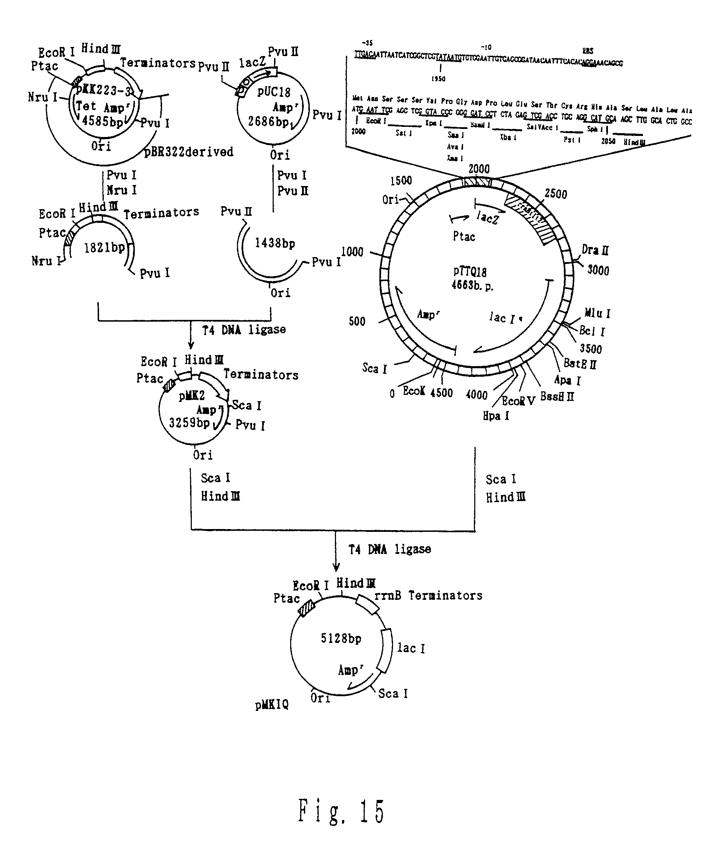

As shown in Fig. 15, a DNA fragment including rrnB terminator region and lacI$^q$ fragment was obtained by digesting commercially available plasmid pTTQ18 with restriction enzymes ScaI and HindIII.

On the other hand, plasmid pMK2, which was described in example 1 was cleaved with restriction enzymes ScaI and HindIII and the DNA fragment mentioned above was inserted using T4 DNA ligase.

This plasmid includes DNA fragments of tac promotor region, replication origin (Ori), and a fragment of ampicillin-registance gene. Escherichia coli JM109 was transformed with this plasmid, cultured and screened for the ampicillin registance. Thus, a vector containing these fragments was prepared. The nucleotide sequence of this plasmid was confirmed by the Sanger method. This plasmid was named pMKIQ.

Next, the above mentioned plasmid was isolated and cleaved with a restriction enzyme EcoRI, as shown in Fig. 15, and 5′ phosphate residue was removed by the treatment with alkaline phosphatase.

On the other hand, DNA coding amino acid sequence of 2 fusion proteins obtained in (1) was inserted

9

into a cleaved site and connected using T4 DNA ligase. Escherichia coli JM109 was transformed with this plasmid and screened folr the ampicillin resistance and the increase in the number of copies. This cloned plasmid was named pIQAKTGFα2.

(3) expression of human TGFα fusion protein

Escherichia coli JM109 , transformed with plasmid pIQAKTGFα2 was cultured in LB medium (bacto-trypton 10 g/l, Bacto-yeast-extract 5g/l, NaCl 10g/l, pH7.5) containing 50 μg/ml of ampicillin. After the growth of Escherichia coli , IPTG was added and the culture continued overnight. Cells was collected by centrifugation and disrupted. The fusion protein comprising a polypeptide N-terminus - 65th of porcine adenylate kinase and TGFα was obtained. The expression of fusion protein was confirmed by SDS-PAGE. This revealed that the fusion protein was expressed in a large quantity reaching 20% of total bacterial body protein. Thus obtained fusion protein was treated with cyanogen-bromide and TGFα was obtained. The production of TGFα was 50 mg from 1 l of culture medium.

The strain transformed with the plasmid pIQAKTGFα2 was deposited to the Fermentation Research Institute as Escherichia coli JM109 pIQAKTGFα2 with deposition No. FERM BP2612.

## Claims

1, An expression vector for a polypeptide chosen from a group composed of a platelet factor 4 and TGFα, which comprises (i) a DNA fragment of tac promotor; (ii) replication origin (Ori) of plasmid pUC18; (iii) a nucleotide sequence coding all or a part of amino acid sequence of a polypeptide chosen from a group composed of human angiogenin and porcine adenylate kinase; and (iv) a nucleotide sequence coding amino acid sequence of a polypeptide chosen from a group composed of platelet factor 4 and transforming growth factor type α (TGFα), and which is designed to express a fusion protein of all or a part of a polypeptide chosen from a group composed of human angiogenin and porcine adenylate kinase with a polypeptide chosen from a group composed of platelet factor 4 and TGFα.

2, An expression vector of claim 1, which expresses a fusion protein of all or a part of amino acid sequence of human angiogenin with platelet factor 4.

3, An expression vector of claim 1, which expresses a fusion protein of all or apart of amino acid sequence of porcine adenylate kinase with TGFα.

4, An expression vector of claim 2, which is named pMAPF4.

5, An expression vector of claim 3, which is named pMAKTGFα.

6, An expression vector of claim 3, which is named pIQAKTGFα.

7, A fusion protein comprising all or a part of amino acid sequence of a polypeptide chosen from a group composed of human angiogenin and pocine adenylate kinase, and a polypeptide chosen from a group composed of platelet factor 4 and TGFα.

8, An Escherichia coli transformed with the vector of claim 1.

9, The Escherichia coli of claim 8, which has characteristics of E. coli JM109 pMAPF4 (FERM BP2610).

10, The Escherichia coli of claim 8, which has characteris of E. coli JM109 pMAKTGFα (FERM BP2611).

11, The Escherichia coli of claim 8, which has characteristicsof E. coli JM109 pIQAKTGFα2 (FERM BP2612).

Glu Ala Glu Glu Asp Gly Asp Leu Gln Cys Leu Cys Val Lys

Thr Thr Ser Gln Val Arg Pro Arg His Ile Thr Ser Leu Glu Val Ile Lys Ala

Gly Pro His Cys Pro Thr Ala Gln Leu Ile Ala Thr Leu Lys Asp Gly Arg Lys

Ile Cys Leu Asp Leu Gln Ala Pro Leu Tyr Lys Lys Ile Ile Lys Lys Leu Leu

Glu Ser

# Fig. 1

```
        10         20         30         40         50
AATTCGTTAA CATGGAAGCT GAAGAAGACG GTGACCTGCA ATGCCTGTGC
    GCAATT GTACCTTCGA CTTCTTCTGC CACTGGACGT TACGGACACG


        60         70         80         90        100
GTTAAGACCA CTTCTCAGGT ACGTCCGCGT CACATCACTT CTCTGGAAGT
CAATTCTGGT GAAGAGTCCA TGCAGGCGCA GTGTAGTGAA GAGACCTTCA


       110        120        130        140        150
AATCAAAGCT GGTCCGCACT GTCCGACTGC TCAGCTGATC GCTACTCTGA
TTAGTTTCGA CCAGGCGTGA CAGGCTGACG AGTCGACTAG CGATGAGACT


       160        170        180        190        200
AAGACGGTCG TAAAATCTGT CTAGACCTGC AAGCTCCACT GTACAAGAAG
TTCTGCCAGC ATTTTAGACA GATCTGGACG TTCGAGGTGA CATGTTCTTC


       210        220        230        240
ATCATCAAAA AACTGCTGGA ATCTTAATGA CTGCAGAAGC TTG
TAGTAGTTTT TTGACGACCT TAGAATTACT GACGTCTTCG AACTTAA
```

# Fig. 2

```
          -35                                  -10
5′ -TGT TGA CAA TTA ATC ATC GGC TCG TAT AAT GTG TGG AAT TGT GAG
    ACA ACT GTT AAT TAG TAG CCG AGC ATA TTA CAC ACC TTA ACA CTC


                                    S/D     EcoR I
    CGG ATA ACA ATT TCA CAC AGG AAA CAG AAT TC  -3′
    GCC TAT TGT TAA AGT GTG TCC TTT GTC TTA A
```

# Fig. 3

EP 0 423 641 A2

```
         10         20         30         40         50         60
EcoR I    |          |          |          |          |          |
AATTCATGC AGGATAACTC TAGATACACC CACTTCCTGA CCCAGCACTA CGATG CGAAA
  GTACG TCCTATTGAG ATCTATGTGG GTGAAGGACT GGGTCGTGAT GCTAC GCTTT


         70         80         90        100        110        120
          |          |          |          |          |          |
CCGCA GGGCC GCGACGATCG CTACTGTGAA TCGATCATGC GGCGCCGCGG CCTGACGTCA
GGCGT CCCGG CGCTGCTAGC GATGACACTT AGCTAGTACG CCGCGGCGCC GGACTGCAGT


        130        140        150        160        170        180
          |          |          |          |          |          |
CCGTGCAAAG ATATCAACAC CTTCATCCAT GGCAACAAAC GCTCGATCAA AGCGATCTGT
GGCACGTTTC TATAGTTGTG GAAGTAGGTA CCGTTGTTTG CGAGCTAGTT TCGCTAGACA


        190        200        210        220        230        240
          |          |          |          |          |   HindⅢ  |
GAAAACAAAA ACGGCAACCC GCATCGCGAA AACCTGCGCA TTTCGAAATC A AGCTTCCAG
CTTTTGTTTT TGCCGTTGGG CGTAGCGCTT TTGGACGCGT AAAGCTTTAG TTCGA AGGTC


        250        260        270        280        290        300
          |          |          |          |          |          |
GTTACCACTT GCAAACTGCA TGGCGGATCC CCGTGGCCAC CGTGCCAGTA CCGCGCGACT
CAATGGTGAA CGTTTGACGT ACCGCCTAGG GGCACCGGTG GCACGGTCAT GGCGCGCTGA


        310        320        330        340        350        360
          |          |          |          |          |          |
GCAGGCTTCC GGAACGTTGT TGTTGCATGC CAAAACGGCC TGCCGGTGCA CCTTGATCAG
CGTCCGAAGG CCTTGCAACA ACAACGTACG CTTTTGCCGG ACGGCCACGT GGAACTAGTC


        370        380
          |          |        EcoR I
TCAATCTTCC GTCGTCCGTA ATG
AGTTAGAAGG CAGCAGGCAT TACTTAA
```

Fig. 4

14

Fig. 5

Fig. 6

Fig. 7

PF4 gene

Klenow

```
|AATTCGT|ATG|············|GTTAA
 TTAAGCA|TAC|············CAATT|
```

Klenow        initiation
              codon

Nru I site

```
TCG↓CGA        blunt end
AGC↑GCT
```

Nru I
HindⅢ
EcoR I        EcoR I
Ptac
h-AGN        rrnB
             Terminators
Amp[r]
Ori
pMAGN1

Ligation

EcoR I        PF4 gene
Ptac    EcoR I
              EcoR I
              rrnB
              Terminators
Amp[r]
Ori
pMAPF4

expression vector for
PF4 fusion protein

# Fig. 8

```
                    Hind Ⅲ
           5′ -AGC TTG ATG GTT GTT TCT CAT TTC AAC GAC
           3′ -       AC TAC CAA CAA AGA GTA AAG TTG CTG
                          Met Val Val Ser His Phe Asn Asp


TGC CCG GAC TCT CAT ACT CAG TTC TGC TTC CAT GGT ACC TGC CGT
ACG GGC CTG AGA GTA TGA GTC AAG ACG AAG GTA CCA TGG ACG GCA
Cys Pro Asp Ser His Thr Gln Phe Cys Phe His Gly Thr Cys Arg


TTC CTG GTT CAG GAA GAC AAA CCG GCA TGC GTT TGT CAT TCT GGT
AAG GAC CAA GTC CTT CTG TTT GGC CGT ACG CAA ACA GTA AGA CCA
Phe Leu Val Gln Glu Asp Lys Pro Ala Cys Val Cys His Ser Gly


TAC GTT GGT GCT CGT TGC GAA CAT GCA GAT CTG CTG GCT TAG TAA
ATG CAA CCA CGA GCA ACG CTT GTA CGT CTA GAC GAC CGA ATC ATT
Tyr Val Gly Ala Arg Cys Glu His Ala Asp Leu Leu Ala


           EcoR I
GGA TCC TAA G       -3′
CCT AGG ATT CTTAA -5′
```

# Fig. 9

```
Pvu II                                    30        Taq I     Hpa I            60
CTG GCA AAT ATT CTG AAA TGA GCT GTT GAC AAT TAA TCA TCG AAC TAG TTA ACT AGT ACG CAA
GAC CGT TTA TAA GAC TTT ACT CGA CAA CTG TTA ATT AGT AGC TTG ATC AAT TGA TCA TGC GTT
                                    -35                              -10


                              90                                      120
GTT CAC GTA AAA AGG GTA TCG ACA ATG AAA GCA ATT TTC GTA CTG AAA GGT TCA CTG GAC AGA
CAA GTG CAT TTT TCC CAT AGC TGT TAC TTT CGT TAA AAG CAT GAC TTT CCA AGT GAC CTG TCT


          Hind III           BamH I       EcoR I
GAT CTA AGC TTG TCT AGA GGA TCC CGG GAA TTC
CTA GAT TCG AAC AGA TCT CCT AGG GCC CTT AAG
Bg III               Xba I        Sma I
```

# Fig. 10

GCACTCGCACCCCTCCGCCAGAGCGCTGACTCAGCTCCCCGGACCGCGGCAGG

ATG GAA GAG AAG CTG AAG AAA AGC AAG ATC ATC TTT GTG GTG GGC
Met Glu Glu Lys Leu Lys Lys Ser Lys Ile Ile Phe Val Val Gly

GGG CCC GGC TCG GGG AAG GGC ACC CAG TGT GAG AAG ATT GTC CAG
Gly Pro Gly Ser Gly Lys Gly Thr Gln Cys Glu Lys Ile Val Gln

AAG TAC GGC TAC ACC CAC CTC TCC ACC GGG GAC CTC CTG CGG GCC
Lys Tyr Gly Tyr Thr His Leu Ser Thr Gly Asp Leu Leu Arg Ala

GAG GTC AGC TCG GGC TCG GCC AGG GGC AAG ATG CTG TCG GAA ATC
Glu Val Ser Ser Gly Ser Ala Arg Gly Lys Met Leu Ser Glu Ile
                         Pvu II
ATG GAG AAG GGG CAG|CTG GTG CCA CTG GAG ACA GTG TTG GAC ATG
Met Glu Lys Gly Gln↓Leu Val Pro Leu Glu Thr Val Leu Asp Met
                  Nco I
CTC CGA GAC GCC ATG GTG GCC AAA GTA GAT ACT TCC AAA GGC TTC
Leu Arg Asp Ala Met↓Val Ala Lys Val Asp Thr Ser Lys Gly Phe
                      Sma I
CTG ATC GAC GGC TAC CCC CGG GAG GTG AAG CAG GGG GAA GAG TTT
Leu Ile Asp Gly Tyr Pro Arg↓Glu Val Lys Gln Gly Glu Glu Phe

GAG CGG AAG ATC GGA CAG CCC ACA CTG CTG CTG TAT GTG GAC GCG
Glu Arg Lys Ile Gly Gln Pro Thr Leu Leu Leu Tyr Val Asp Ala

GGC CCT GAG ACC ATG ACC AAG CGG CTC CTG AAG CGC GGA GAG ACC
Gly Pro Glu Thr Met Thr Lys Arg Leu Leu Lys Arg Gly Glu Thr

AGT GGG CGC GTG GAC GAC AAC GAA GAG ACC ATC AAG AAG CGG CTG
Ser Gly Arg Val Asp Asp Asn Glu Glu Thr Ile Lys Lys Arg Leu

GAG ACC TAC TAC AAG GCC ACA GAG CCC GTC ATT GCC TTC TAC GAG
Glu Thr Tyr Tyr Lys Ala Thr Glu Pro Val Ile Ala Phe Tyr Glu

AAA CGT GGC ATC GTT CGC AAG GTC AAT GCC GAA GGC TCC GTG GAC
Lys Arg Gly Ile Val Arg Lys Val Asn Ala Glu Gly Ser Val Asp

GAT GTC TTC TCG CAG GTG TGC ACC CAC TTG GAC ACC CTC AAG TAG
Asp Val Phe Ser Gln Val Cys Thr His Leu Asp Thr Leu Lys

CCAAGCTGGAGCCCCTTCCCCTGCTCAGAGCCCCCGCCCTGCCCTCATCCTGACTGGGC
CCTCGCCCCTCGCTCAGCGGCGGCGCGCCCTGCCCGGCTGGAGCACAGGCAGAGGAAGC
CACTTTATCCTGTTTTCATGGACAGCCGAACACTAAAGGAATTTCCAAGGACATTCGAT

Fig. 11

Fig. 12

EcoR I  Pvu II  Hind III
Ptac
Amp'
Ori
pMKAK3

TrpLE  Hind III  EcoR I
Ptrp
Amp'
Ori
pTZT

Pvu II digest
CIP treatment

EcoR I -Hind III digest

Hind II  EcoR I
TGF α Gene

Klenow reaction

Ligation

ADK
65 | 66  67
Gln | Leu  Val
CAG | CTG  GTG
GTC ↓ GAC  CAC
pVU II

pMKAK3

TGF α
Met  Val  Val
AAGCTTG ATG GTT GTT……GAATTC
TTCGAAC TAC CAA CAA……CTTAAG
Hind III →Klenow    EcoR I →Klenow

EcoR I  TGF α  EcoR I regeneration
Ptac    Hind III
Amp'
rrnBT
Ori
pMAKTGF α

Fig. 13

23

pMAKTGFα

EcoR I digest

E ———————— E

TGFα

pMKIQ

EcoR I digest

CIP treatment

Ligation

EcoR I    TGFα

EcoR I

Ptac    EcoR I

rrnBT

Ampr

Ori

pIQAKTGFα W

Fig. 14

F i g. 15

25